# EUROPEAN PATENT APPLICATION

(11) **EP 2 801 273 A2**
(43) Date of publication of application: **12.11.2014**
(21) Application number: 14165912.8
(22) Date of filing: 24.04.2014
(51) Int. Cl.: A24F 47/00, A61M 15/06

(54) **Atomizing device and electronic cigarette having same**

(30) Priority: 07.05.2013 CN 201320245260 U
(71) Applicant: Shenzhen First Union Technology Co., Ltd., Shenzhen Guangdong 518104 (CN)
(72) Inventor: Li, Yonghai, 518104 Shenzhen (CN); Xu, Zhongli, 518104 Shenzhen (CN)
(74) Representative: ProI European Patent Attorneys

(57) **Abstract**

An atomizing device (100) of an electronic cigarette includes an atomizing sleeve (1); an oil reserving member (5) arranged in the atomizing sleeve and configured for reserving a tobacco oil; an air pipe (41) extending through the oil reserving member, a porous body (42) and a heating coil (43). The air pipe has a clamping portion (411) formed thereon, and the porous body is clamped by the clamping portion and extending into the oil reserving member to absorb the tobacco oil. The heating coil wraps around the porous body and is configured for heating and atomizing the tobacco oil on the porous body. An electronic cigarette is also provided, which includes the atomizing device and a power supply configured for powering the atomizing device.

## Description

### BACKGROUND

### 1. Technical Field

The present invention relates to atomizing devices, and particularly to an atomizing device and an electronic cigarette having the atomizing device.

### 2. Description of Related Art

Electronic cigarettes are similar to conventional cigarettes in appearance and taste, but less harmful to human's health, so that electronic cigarettes are widely used for helping people to quit smoke. Atomizing devices are key components of electronic cigarettes. A typical atomizing device mainly includes an atomizing sleeve, an air pipe arranged in the atomizing sleeve, a porous body arranged on an end of the air pipe and configured for absorbing tobacco oil, and a heating coil wrapping around the porous body and connected to other conductive components.

In the above atomizing device, shortcomings may occur: first, the porous body and the heating coil are not firmly supported by other components, so that in case of the electronic cigarette falls off, the porous body may leave the original position, thus cannot well absorb the tobacco oil. Second, the air pipe is fixed to another component by glue, resulting a lower efficiency of the assembly. Third, as the porous body is arranged on an end of the air pipe, to apply an oil reserving member, such as a cotton layer on both the air pipe and the porous body is difficult.

What is needed, therefore, is an atomizing device and an electronic cigarette which can overcome the above shortcomings.

### BRI EF DESCRIPTION OF THE DRAWINGS

Many aspects of the present atomizing device and electronic cigarette can be better understood with reference to the following drawings. The components in the drawings are not necessarily drawn to scale, the emphasis instead being placed upon clearly illustrating the principles of the present atomizing device and electronic cigarette. Moreover, in the drawings, like reference numerals designate corresponding parts throughout the several views.
FIG. 1 is a schematic cross sectional view of an atomizing device of an electronic cigarette in accordance with a first embodiment.
FIG. 2 is an exploded view of the atomizing device of FIG. 1.
FIG. 3 is a schematic view of an air pipe in accordance with a second embodi ment.
FIG. 4 shows a glass fiber core is assembled to the air pipe of FIG. 3.
FIG. 5 is a schematic view of a fixing sleeve used in the first and second embodiments.
FIG. 6 is a schematic cross sectional view of an electronic cigarette in accordance with a third embodiment.
FIG. 7 is a schematic cross sectional view of an electronic cigarette in accordance with a fourth embodiment.

### DETAILED DESCRIPTION

Embodiments of the present atomizing device and electronic cigarette will now be described in detail below and with references to the drawings.

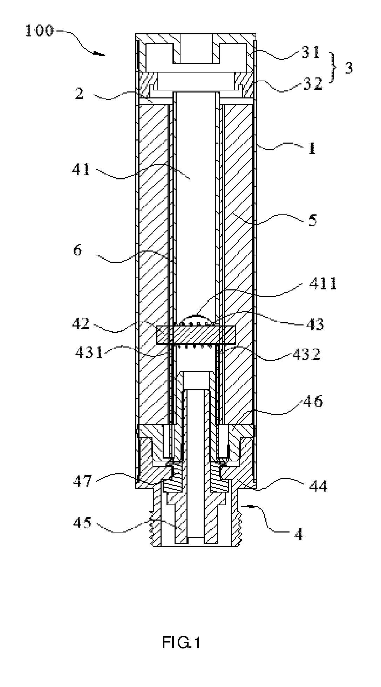

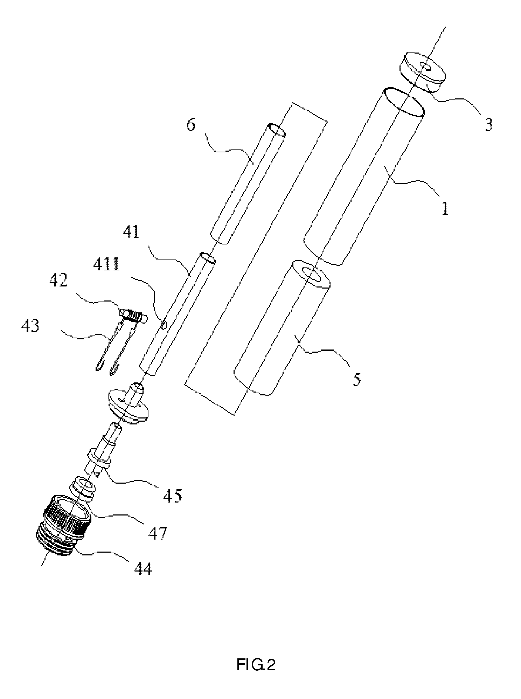

Referring to FIGs. 1 and 2, an atomizing device 100 of an electronic cigarette in accordance with a first embodiment is provided. The atomizing device 100 mainly includes an atomizing sleeve 1, a mouthpiece assembly 3 arranged at an upper end of the atomizing sleeve 1, and an atomizing assembly 4 arranged at a lower end of the atomizing sleeve 1. The atomizing sleeve 1 includes an oil reserving space 2 defined between the mouthpiece assembly 3 and the atomizing assembly 4, and an oil reserving member 5 received in the oil reserving space 2 and configured for reserving tobacco oil filled in oil reserving space 2. The oil reserving member 5 can be rolls of a fiber cotton or other materiel capable of reserving oil.

The atomizing assembly 4 includes an air pipe 41, a glass fiber core 42, a heating coil 43 wrapping around the glass fiber core 42, a screw sleeve 44 fixed at the lower end of the atomizing sleeve 1, a tubular electrode 45 arranged in the screw sleeve 44, and an insulated ring 47 arranged between the screw sleeve 44 and the tubular electrode 45. The heating coil 43 has two ends 431,432 with the end 431 electrically connected to the tubular electrode 45, and the other end 432 electrically connected to the screw sleeve 44. The air pipe 41 extends through the oil reserving member 5, with one end of the air pipe 41 connected to the mouthpiece assembly 3 and the other end of the air pipe 41 connected to the screw sleeve 44. The glass fiber core 42 is retained by the air pipe 41 and extends to the oil reserving member 5 to absorb the tobacco oil.

In particular, the air pipe 41 has a clamping portion 411 formed in a wall of the air pipe 41. In the present embodiment, the clamping portion 411 is a cutout formed in the air pipe 41 with a gap formed in a wall of the air pipe 41. The cutout is tightly adapted for the glass fiber core 42. The glass fiber core 42 is clamped by the clamping portion 411, with two ends of the fire core 42 extending to outside of the air pipe 41 and in contact with the oil reserving member 5. The two ends 431,432 of the heating coil 43 extend to outside of the air pipe 41 to be electrically connected to the tubular electrode 45 and the screw sleeve 44, respectively.

After the glass fiber core 42 is assembled into the air pipe 41, a cotton layer 6 is applied on a peripheral wall of the air pipe 41, with the two ends of the glass fiber core 42 in contact with the cotton layer 6. The oil reserving member 5 then surrounds the cotton layer 6, so that the tobacco oil in the oil reserving member 5 can be absorbed by the cotton layer 6, and finally absorbed by the glass fiber core 42. In this way, the tobacco oil can be heated and atomized by the heating coil 43. The cotton layer 6 helps the tobacco oil reserved in the oil reserving member 5 to be well absorbed by the glass fiber core 42, and thus improve atomization of the tobacco oil.

The mouthpiece assembly 3 includes a mouthpiece cap 31 and a liquid stopper 32. The liquid stopper 32 is arranged on the oil reserving member 5 to stop the tobacco oil in the oil reserving member 5 to flow toward the mouthpiece cap 31.

The screw sleeve 44 may be outer-screw sleeve or inner-screw sleeve, and is configured to be threadedly connected to a power pole to form an electronic cigarette. The screw makes the assembly and disassembly easy.

From the above description of the atomizing device 100, the glass fiber core 42 is clamped by the clamping portion 411, the heating coil 43 wraps on the glass fiber core 42, the cotton layer 6 and the oil reserving member 5 then surrounds the glass fiber core 42. By this way, the glass fiber core 42 is tightly held, and would not leap in case of the atomizing device 100 falls off.

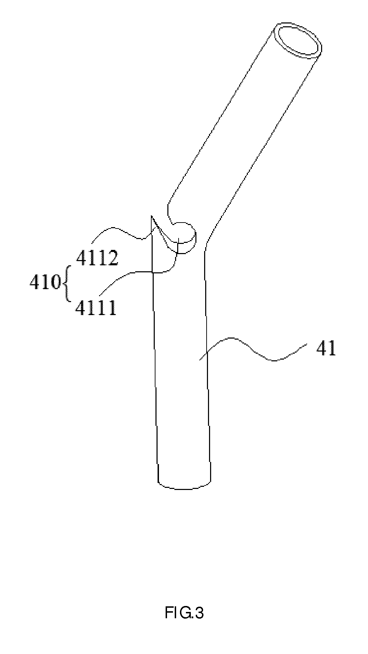

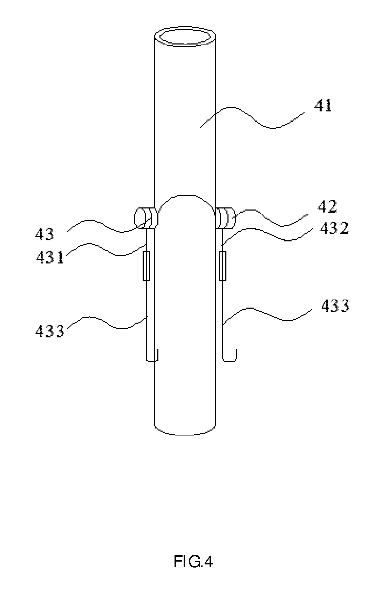

Referring to FIGs. 3 and 4, in a second embodiment, a clamping portion 410 of an air pipe 40 includes a cutout 4111 substantially in a circular through hole shape, and a tip portion 4112 configured as a gate of the cutout 4111. In assembling the glass fiber core 42, the air pipe 40 can be flexed at the clamping portion 410, so that the tip portion 4112 and the cutout 4111 are open up for receiving the glass fiber core 42. The two ends 431,432 of the heating coil 43 extend to outside of the air pipe 41. After that, the air pipe 41 can get back due to its flexible characteristics, and thus the glass fiber core 42 is tightly clamped, with a central axis of the glass fiber core 42 being substantially perpendicular to that of the air pipe 41. The tip portion 4112 can make the glass fiber core 42 more steadily held by the clamping portion 410.

It is understood that the glass fiber core 42 can be replaced by other porous body, such as ceramic.

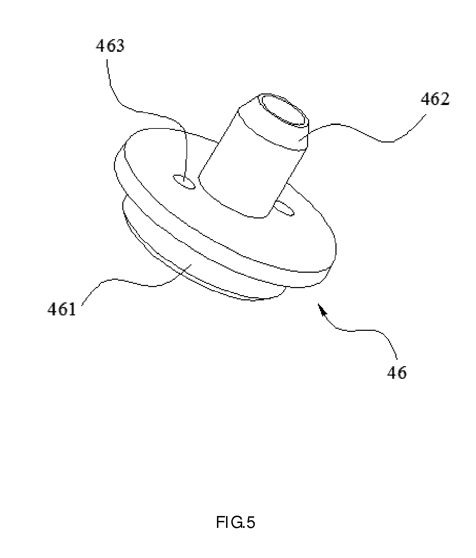

Referring to FIG. 5 and again to FIG. 1, a fixing sleeve 46 is provided on the screw sleeve 44. The fixing sleeve 46 includes two small holes 463 for receiving the two ends 431,432 of the heating coil 43, respectively. The small holes 463 can help separating the two ends 431, 432. The fixing sleeve 46 further includes a hollow projection 461 extending downwards thereof, and a hollow column 462 extending upwards thereof. The projection 461 is engaged in the screw sleeve 44, and the column 462 is engaged in the air pipe 41, so that the fixing sleeve 46 and together with the air pipe 41 is fixed.

The tubular electrode 45 is inserted into the projection 461 and the column 462, thus the end 431 of the heating coil 43, which is received in one of the small holes 463 is electrically connected to the tubular electrode 45; and the other end 432 of the heating coil 43, which is received in the other one of the small holes 463 is electrically connected to the screw sleeve 44. The tubular electrode 45 and the screw sleeve 44 are configured as positive end and negative end. In order to avoid the tubular electrode 45 contacts the screw sleeve 44, an insulated ring 47 is arranged between the tubular electrode 45 and the screw sleeve 44.

The screw sleeve 44, the tubular electrode 45, the fixing sleeve 46, and the air pipe 41 are in communication with each other, thereby making air flow smooth.

In particular, the two ends 431,432 of the heating coil 43 are electrically connected to the screw sleeve 44 and the tubular electrode 45 by conductive wires 433. The conductive wires 433 can be made of materials such as copper, which has lower resistance than that of the heating coil 43. The conductive wires 433 can be connected to the heating coil 43 by riveting instead of wedding, so that a peculiar smell due to wedding can be avoided.

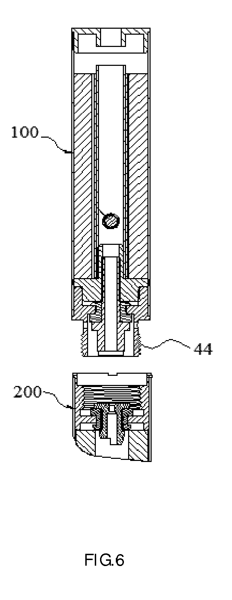

Referring to FIG. 6, an electronic cigarette in accordance with a third embodiment is provided. The electronic cigarette includes an atomizing device 100 and a power supply 200 configured to power the atomizing device 100. In the present embodiment, the power supply 200 is detachably connected to the atomizing device 100.

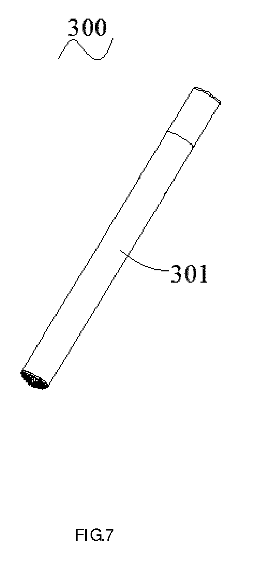

Referring to FIG. 7, an electronic cigarette 300 in accordance with a fourth embodiment is provided. In the present embodiment, the electronic cigarette is a disposable electronic cigarette, with the power supply and the atomizing device received in a same shell 301.

It is understood that the above-described embodiments are intended to illustrate rather than limit the disclosure. Variations may be made to the embodiments and methods without departing from the spirit of the disclosure. Accordingly, it is appropriate that the appended claims be construed broadly and in a manner consistent with the scope of the disclosure.

## Claims

1. An atomizing device of an electronic cigarette, comprising:
an atomizing sleeve;
an oil reserving member arranged in the atomizing sleeve and configured for reserving a tobacco oil;
an air pipe extending through the oil reserving member, the air pipe having a clamping portion formed thereon;
a porous body clamped by the clamping portion and extending into the oil reserving member to absorb the tobacco oil; and
a heating coil wrapping around the porous body and configured for heating and atomizing the tobacco oil on the porous body.

2. The atomizing device of claim 1, wherein the oil reserving member includes a roll of a fiber cotton.

3. The atomizing device of claim 1, wherein the clamping portion is a cutout formed in a wall of the air pipe.

4. The atomizing device of claim 1, wherein the clamping portion includes a cutout and a tip portion each formed in a wall of the air pipe.

5. The atomizing device of claim 1, wherein the porous body is a glass fiber core which absorbs the tobacco oil by capillary.

6. The atomizing device of claim 1, further comprising a screw sleeve and a tubular electrode arranged at an end of the atomizing sleeve, the tubular electrode being spaced apart the screw sleeve by an insulated member, and two ends of the heating coil being electrically connected to the screw sleeve and the tubular electrode respectively.

7. The atomizing device of claim 6, wherein the two ends of the heating coil are electrically connected to the screw sleeve and the tubular electrode respectively each by a conductive wire, and the two ends of the heating coil and the conductive wires are connected by riveting.

8. The atomizing device of claim 7, further comprising a fixing sleeve having a projection extending toward a first end thereof, and a column extend toward an opposite second end thereof, the projection engaged with the screw sleeve, and the column engaged in the air pipe.

9. The atomizing device of claim 8, wherein the fixing sleeve comprises two holes for receiving the conductive wires.

10. The atomizing device of claim 8, wherein a material of the fixing sleeve is selected from silicon dioxide and plastic.

11. An atomizing device of an electronic cigarette, comprising:
an atomizing sleeve;
an oil reserving member arranged in the atomizing sleeve and configured for reserving a tobacco oil;
an air pipe extending through the oil reserving member, the air pipe having a clamping portion formed thereon;
a porous body clamped by the clamping portion and extending into the oil reserving member to absorb the tobacco oil, a central axis of the porous body being substantially perpendicular to that of the air pipe; and
a heating coil wrapping around the porous body and configured for heating and atomizing the tobacco oil on the porous body.

12. An electronic cigarette comprising an atomizing device and a power supply configured for powering the atomizing device, wherein the atomizing device comprising:
an atomizing sleeve;
an oil reserving member arranged in the atomizing sleeve and configured for reserving a tobacco oil;
an air pipe extending through the oil reserving member, the air pipe having a clamping portion formed thereon;
a porous body clamped by the clamping portion and extending into the oil reserving member to absorb the tobacco oil; and
a heating coil wrapping around the porous body and configured for heating and atomizing the tobacco oil on the porous body.

13. The electronic cigarette of claim 12, wherein the power supply is detachably connected to the atomizing device.

14. The electronic cigarette of claim 12, further comprising a shell receiving the atomizing device and the power supply therein.
